# EUROPEAN PATENT SPECIFICATION

(11) **EP 3 790 520 B1**
(45) Date of publication and mention of the grant of the patent: **25.03.2026**
(21) Application number: 19725047.5
(22) Date of filing: 08.05.2019
(51) Int. Cl.: A61F 13/66, A61F 13/49, A41B 9/00, A41B 9/12, A61F 13/15

(54) **MULTI-PURPOSE DISPOSABLE UNDERWEAR**
WEGWERFBARE MEHRZWECKUNTERWÄSCHE
SOUS-VÊTEMENT JETABLE POLYVALENT

(30) Priority: 08.05.2018 US 201862668339 P; 17.07.2018 NL 2021325
(43) Date of publication of application: 17.03.2021
(73) Proprietor: Drylock Technologies NV, 9240 Zele (BE)
(72) Inventor: GLAUG, Frank, Chester Springs, Pennsylvania 19425 (US); ANDERSEN, Karl, River Falls, Wisconsin 54022 (US); BORRERO, Ricardo, Altoon, Wisconsin 54720 (US)
(74) Representative: Arnold & Siedsma
(86) International application number: PCT/EP2019/061856
(87) International publication number: WO 2019/215249

(56) References cited:
- WO-A1-2017/158185
- WO-A2-02/34182
- WO-A2-2009/117492

## Description

### Field

The present disclosure relatesto a method for making a disposable absorbent product and a disposable garment..

### Background

There are several types of commercially available products for the absorption of bodily fluids. Such absorbent products are available in different types, designs, and dimensions, each one having one or more unique features. For example, training pants, baby diapers, adult diapers, and incontinence guards are products designed for the containment of urine and excrement. There are other types of disposable absorbent articles, such as feminine hygiene products (e.g., heavy and light incontinence pads, pantyliners, etc.) that are designed to contain and absorb urine and/or menses by female wearers. Another type of absorbent article includes underpads configured to absorb and collect body fluid discharge from a person who may be generally confined to a bed or chair, or may otherwise be immobilized.

Current absorbent products in the form of underwear, training pants, or diapers, generally suffer from poor design. For example, in order to hold the product close to the wearer's body so that it does not fall down, elastic strands or other features are usually provided around leg openings and/or waist openings of the absorbent product. However, this type of construction often leads to discomfort to the wearer from the rubbing of the overlapping components and/or seams holding the components together. Additionally, the elastic elements around the leg and waist openings used to hold the garment against the wearer often contribute to leakage. The leakage is due to the gapping between the article and the wearer's body caused by the elastic gatherings, the gapping providing an inconsistent seal. Furthermore, many current absorbent underwear products contribute to a bulky appearance when worn.

Accordingly, improperly-fit absorbent garments can lead to a number of different issues for the wearer of the garment. For example, an improperly-fit garment can be uncomfortable, can adversely affect the wearer's mobility (actual or perceived), and there can be an increased chance of leakage. This in undesirable to a wearer and can be cause for embarrassment and shame. Thus, there exists a need to minimize leakage at the openings and providing a smooth contoured fit to the body of the wearer.

### Summary

The present invention is set out in the appended set of claims.

The present disclosure relates generally to disposable article. More specifically, the present disclosures relates to a disposable undergarment that includes a stretchable product chassis configured to serve as a basis for a variety of disposable articles, including, but not limited to, disposable underwear without an absorbent core, disposable underwear with an absorbent core designed for menstrual use, and disposable underwear or training pant with an absorbent core designed for incontinence use (adult and child). The product chassis is comprised of multiple sections coupled to one another. For example, in one embodiment, a product chassis may generally include three main sections, which includes a front section, a rear section, and a crotch section. Each section of the product chassis generally includes at least two substrate layers containing an elastomeric material disposed there between. In some embodiments, an absorbent core may be disposed between the one or two substrate layers. However, in other embodiments, an absorbent core can be applied on top of the two substrate layers.

The inclusion of an elastomeric material, such as a stretch adhesive, in the construction of the sections of the product chassis results in improved securement of the article to a wearer for reducing the risk of leakage and further improving comfort for the wearer. In particular, each section is designed to stretch in a desired direction, as set out in the appended set of claims, thereby resulting in improved securement of the undergarment to a wearer for optimizing fit and comfort for the wearer, while reducing the risk of leakage as well as reducing the amount overall material required for producing the undergarment. Furthermore, the inclusion of an elastomeric material can provide a smooth contoured fit to a wearer, thereby improving appearance and self-confidence for the wearer.

In one embodiment, the elastomeric material is a stretch film that can be unwound in the machine direction (MD) along an absorbent article assembly line. The stretchable film can impart a stretchable characteristic to an assembly, for example, after the film is stretched and bonded between two layers of hydrophobic nonwoven materials (under tension), allowing the nonwoven layers to retract after the product is relaxed (no tension). In an example, a stretchable film can be used in place of elastic strands, wherein the film can be applied to augment an extension or contraction characteristic of another stretchable material such as elastic strands or stretchable adhesive. Generally, the elastomeric material is configured to stretch in the machine direction, which is the direction in which the stretchable film was applied to form the elastomeric composite for the front and rear sections of the product chassis. As for the crotch section of the product chassis, it will be a prefabricated elastomeric composite which will also be applied in the machine direction. This elastomeric composite is designed to stretch in the cross direction and not stretch in the machine direction. It is easier to control materials that do not stretch in the machine direction on a high-speed manufacturing line.

The elastomeric material and the two substrate layers form elastomeric composites for each given section of the product chassis (i.e., the front, rear, and crotch sections). Each of the sections may be strategically constructed so as to stretch in a specific direction to help maximize the fit on the wearer from a minimum to a maximum range, thereby accommodating the varying shapes and sizes from wearer to wearer. For example, in one embodiment, the front section, which is generally designed to be fitted against the front, or anterior portion, of a wearer, and the rear section, which is generally designed to be fitted against the rear, or posterior portion, of the wearer, may both be constructed so as to stretch in a machine direction (MD), while the crotch section, which is designed to be fitted against the crotch region of the wearer, may be constructed so as to stretch in a cross direction (CD).

By providing different stretching characteristics for each given section, the product chassis provides a superior fitting undergarment with improved comfort, as well as reducing the risk of leakage and, in some cases, provides a much more economical disposable underwear design. For example, because the undergarment lies flat against a wearer's body in at least the three main areas (front section, rear section, and crotch section), the undergarment is about to provide a smooth contoured fit to the body, such that any gaps caused by overlapping materials are reduced or eliminated. By reducing or eliminating gaps between the garment and the body, the undergarment of the present disclosure provides improved leak resistance over prior art constructions. Additionally, the elastomeric composite provides consistent pressure and smooth contact against the leg and waist during wear when it is stretched, such that improved performance against leakage is obtained.

The smooth interface of the undergarment with the body provides improved comfort for the wearer and reduces the risk of skin irritation, particularly around the crotch and waist areas of the consumer, which are some of the most sensitive areas of the body. More specifically, the elastomeric composite design of the present disclosure eliminates the need for individual elastic strands (under high tension) and stiff materials (nonwoven & adhesive) along the leg openings, which are commonly found in current disposable underwear designs. In addition, the elastomeric composite design of the present disclosure allows for a wearer to participate in physical activities that may otherwise cause irritation, such as active motion of the wearer during walking or running exercises, which generally increases the rubbing action of material against the skin. The softer material and lower tension elastic provided by the undergarment of the present disclosure reduces the risk of skin irritation long the leg and crotch area of the consumer.

The use of at least three separately constructed sections (i.e., the front, rear, and crotch section, which are then coupled to one another to form the final undergarment product (via ultrasonic bonding or the like) minimizes waste of what could otherwise be an expensive elastomeric material. In particular, less material is necessarily required to be removed during the manufacturing process, as compared to the amount of material required to be removed if the undergarment was made entirely from single sheets of elastomeric composites.

### Brief Description of the Drawings

Features and advantages of the claimed subject matter will be apparent from the following detailed description of embodiments consistent therewith, which description should be considered with reference to the accompanying drawings.
FIG. 1 is a top plan view of one embodiment of disposable underwear consistent with the present disclosure, illustrating the underwear in an unfolded and unsealed state.
FIG. 2 is a top plan view of the disposable underwear of FIG. 1, illustrating what would constitute typical or normal material waste along leg cut-out sections (via conventional underwear design construction).
FIG. 3 is a top plan view of the disposable underwear of FIG. 1, illustrating reduced material waste along leg cut-out sections.
FIG. 4 is a top plan view of another embodiment of disposable underwear consistent with the present disclosure, illustrating the underwear including an absorbent core and insert provided within at least the crotch section, as well as a stand-up leg gather. The disposable underwear may be useful as a disposable incontinence product.
FIG. 5 is a front view of another embodiment of disposable underwear consistent with the present disclosure, illustrating the underwear in a folded and sealed state and having an anatomically-shaped design.
FIG. 6 is a top plan view of the disposable underwear of FIG. 5 in an unfolded and unsealed state, illustrating reduced material waste along leg cut-out sections.
FIG. 7 is a top plan view of the disposable underwear of FIG. 5 in an unfolded and unsealed state, illustrating what would constitute typical or normal material waste along leg cut-out sections (via conventional underwear design construction).
FIG. 8 is a top plan view of another embodiment of disposable underwear consistent with the present disclosure, illustrating the underwear in an unfolded and unsealed state and having a boxer short shape.
FIGS. 9 and 10 are schematic representations of a method for manufacturing a disposable underwear in accordance with the present disclosure.

For a thorough understanding of the present disclosure, reference should be made to the following detailed description, including the appended claims, in connection with the above-described drawings. Although the present disclosure is described in connection with exemplary embodiments, the disclosure is not intended to be limited to the specific forms set forth herein. It is understood that various omissions and substitutions of equivalents are contemplated as circumstances may suggest or render expedient.

### Detailed Description

The present disclosure relates generally to disposable articles, and, more particularly, to a disposable undergarment including a stretchable product chassis configured to serve as a basis for a variety of disposable articles, including, but not limited to, disposable underwear without an absorbent core, disposable underwear with an absorbent core designed for menstrual use, and disposable underwear or training pant with an absorbent core designed for incontinence use (adult and child). The product chassis is comprised of multiple sections coupled to one another. For example, in one embodiment, a product chassis may generally include three main sections, which includes a front section, a rear section, and a crotch section. Each section of the product chassis generally includes at least two substrate layers containing an elastomeric material disposed there between. In some embodiments, an absorbent core may be disposed between the two substrate layers. However, in other embodiments, an absorbent core can be applied on top of the two substrate layers.

The elastomeric material and the two substrate layers form elastomeric composites for each given section of the product chassis (i.e., the front, rear, and crotch sections). Each of the sections may be strategically constructed so as to stretch in a specific direction to help maximize the fit on the wearer from a minimum to a maximum range, thereby accommodating the varying shapes and sizes from wearer to wearer. For example, in one embodiment, the front section, which is generally designed to be fitted against the front, or anterior portion, of a wearer, and the rear section, which is generally designed to be fitted against the rear, or posterior portion, of the wearer, is constructed so as to stretch in a machine direction (MD), while the crotch section, which is designed to be fitted against the crotch region of the wearer, is constructed so as to stretch in a cross direction (CD).

By providing different stretching characteristics for each given section, the product chassis provides a superior fitting undergarment with improved comfort, as well as reducing the risk of leakage and, in some cases, provides a much more economical disposable underwear design. For example, because the undergarment lies flat against a wearer's body in at least the three main areas (front section, rear section, and crotch section), the undergarment is about to provide a smooth contoured fit to the body, such that any gaps caused by overlapping materials are reduced or eliminated. By reducing or eliminating gaps between the garment and the body, the undergarment of the present disclosure provides improved leak resistance over prior art constructions. Additionally, the elastomeric composite provides consistent pressure and smooth contact against the leg and waist during wear when it is stretched, such that improved performance against leakage is obtained.

The smooth interface of the undergarment with the body provides improved comfort for the wearer and reduces the risk of skin irritation, particularly around the crotch and waist areas of the consumer, which are some of the most sensitive areas of the body. More specifically, the elastomeric composite design of the present disclosure eliminates the need for individual elastic strands (under high tension) and stiff materials (nonwoven & adhesive) along the leg openings, which are commonly found in current disposable underwear designs. In addition, the elastomeric composite design of the present disclosure allows for a wearer to participate in physical activities that may otherwise cause irritation, such as active motion of the wearer during walking or running exercises, which generally increases the rubbing action of material against the skin. The softer material and lower tension elastic provided by the undergarment of the present disclosure reduces the risk of skin irritation long the leg and crotch area of the consumer.

The use of at least three separately constructed sections (i.e., the front, rear, and crotch section, which are then coupled to one another to form the final undergarment product (via ultrasonic bonding or the like) minimizes waste of what could otherwise be an expensive elastomeric material. In particular, less material is necessarily required to be removed during the manufacturing process, as compared to the amount of material required to be removed if the undergarment was made entirely from single sheets of elastomeric composites.

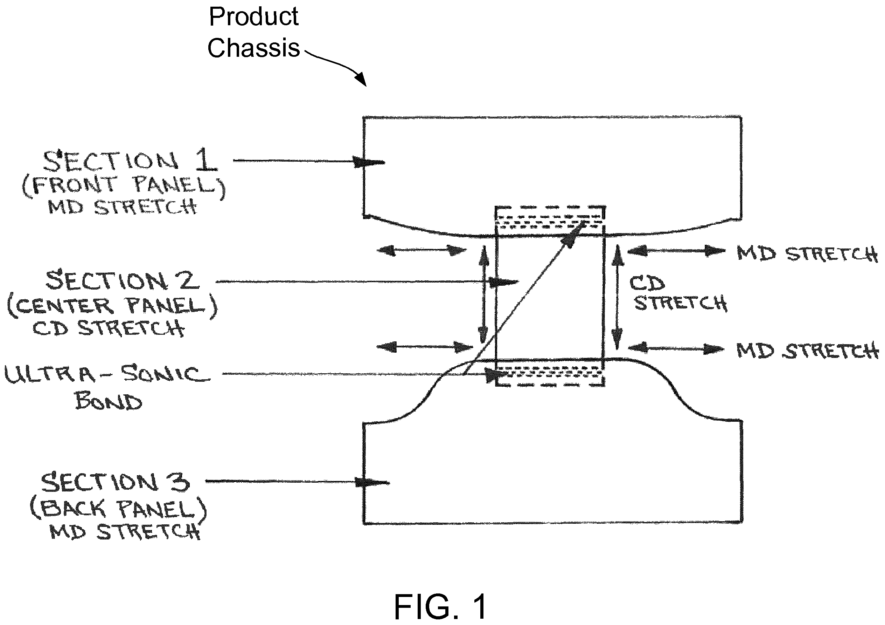

FIG. 1 is a top plan view of one embodiment of disposable underwear consistent with the present disclosure, illustrating the underwear in an unfolded and unsealed state. As shown, the disposable underwear generally includes a stretchable product chassis configured to serve as a basis for a variety of disposable articles, including, but not limited to, disposable underwear without an absorbent core, disposable underwear with an absorbent core designed for menstrual use, and disposable underwear or training pant with an absorbent core designed for incontinence use (adult and child).

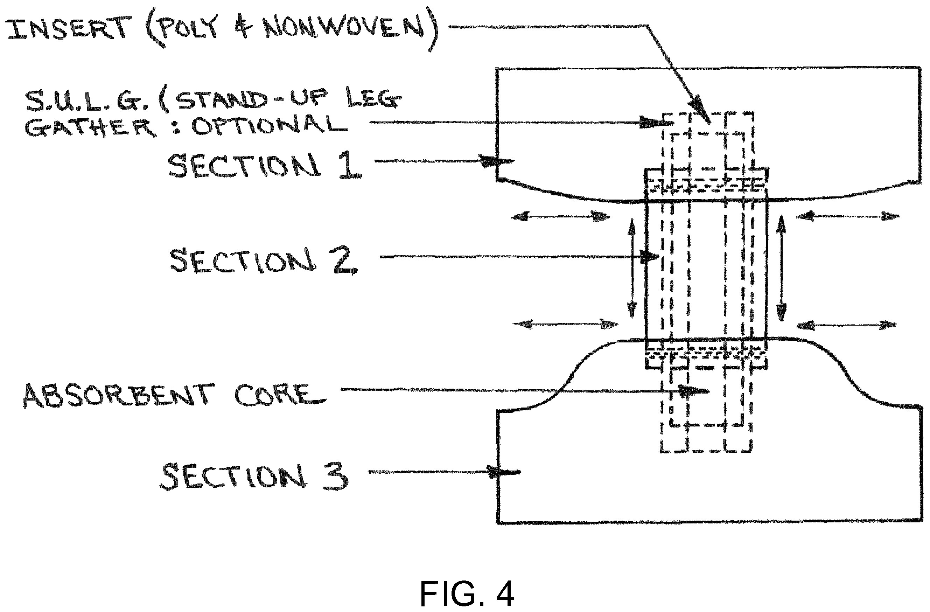

The product chassis is comprised of multiple sections coupled to one another. For example, as shown, the product chassis generally includes three main sections: a front section (section 1); a rear section (section 3); and a crotch section (section 2). The front section is generally designed to be fitted against the front, or anterior portion, of a wearer and the rear section is generally designed to be fitted against the rear, or posterior portion, of the wearer, such that front and rear sections generally oppose one another once fitted to the wearer. The crotch section is positioned between the front and rear sections and coupled to each. In some embodiments, the crotch section may include an insert, such as an absorbent core including a liquid-absorbing material (see FIG. 4). However, in other embodiments, including the embodiment currently shown, the product chassis may be devoid of any absorbent core. Rather, an absorbent core can be added after initial construction of the product chassis. For example, commercially available incontinence pads and pantyliners can be attached along the top sheet.

Each of the front section, rear section, and crotch section are stretchable for providing improved securement of the underwear to a wearer for reducing the risk of leakage and further improving comfort for the wearer, as well as a more smooth contoured fit to a wearer, thereby improving appearance and self-confidence for the wearer.

For example, each section of the product chassis generally includes at least two substrate layers containing an elastomeric material disposed there between. **In** some embodiments, an absorbent core may be disposed between the two substrate layers. However, in other embodiments, an absorbent core can be applied on top of the two substrate layers.

The first substrate may be referred to as a top sheet and is generally configured to contact the wearer's skin and allow fluid from the wearer to flow through it, at least in a direction away from the wearer's skin. **In** some embodiments, the top sheet may include at least one of a nonwoven material, a hydrophilic or partially hydrophilic material, a nonwoven material with a zone-coated surfactant, and a nonwoven and apertured film. One material that can be used for the top sheet is an SBPP (Spunbond Polypropylene) hydrophilic nonwoven, commercially available from Berry Global, located in Charlotte, NC. The second substrate may be referred to as a back sheet and may generally serve as the outermost layer of the product chassis of the underwear. The back sheet is generally formed from a nonwoven material, to provide a more undergarment-like appearance and feel, and as well as a more cost-effective and comfortable alternative to conventional disposable undergarment designs. The fibers in the nonwoven may include, for example, polypropylene, polyethylene, polyester, bi-component (polypropylene & polyethylene or polyester & polyethylene), cotton, cotton blend, viscose, rayon, etc. or a combination of different fibers. The nonwoven web may include, but is not limited to, Spunbond Polypropylene (SBPP), Spunbond-Meltblown-Spunbond (SMS), Thermal-bonded Carded Web, Spunlace, Laminate, or combinations thereof.

The inclusion of an elastomeric material, which comprises a stretch film bonded between two layers of hydrophobic nonwoven materials, in the construction of the sections of the product chassis results in improved securement of the article to a wearer for reducing the risk of leakage and further improving comfort for the wearer. In particular, each section may be designed to stretch in a desired direction, as set out in the appended claims, thereby resulting in improved securement of the undergarment to a wearer for optimizing fit and comfort for the wearer, while reducing the risk of leakage as well as reducing the amount overall material required for producing the undergarment. Furthermore, the inclusion of an elastomeric material can provide a smooth contoured fit to a wearer, thereby improving appearance and self-confidence for the wearer.

In one embodiment, the elastomeric material is a stretch film that is unwound in the machine direction (MD) along an absorbent article assembly line. "Machine direction (MD)", "longitudinal" and the like as used herein are used interchangeably and refer to a direction running parallel to the maximum linear dimension of the structure and includes directions within ±45° of the longitudinal direction.

The stretchable film can impart a stretchable characteristic to an assembly, for example, after the film is stretched and bonded between two layers of hydrophobic nonwoven materials (under tension), allowing the nonwoven layers to retract after the product is relaxed (no tension). In an example, a stretchable film can be used in place of elastic strands and can be applied to augment an extension or contraction characteristic of another stretchable material such as elastic strands or stretchable adhesive. Generally, the elastomeric composite is configured to stretch in the direction in which the stretchable film was applied to form the elastomeric composite. Thus, if the stretchable was applied in a machine direction, then the elastomeric composite will stretch in the machine direction. More specifically, in this embodiment, the elastomeric composite (used for the front and rear sections of the product chassis) will be assembled on the manufacturing machine and stretch in the machine direction. The elastomeric composite used for the crotch (central) sections of the product chassis will be prefabricated, prior to placing on the manufacturing machine. The elastic composite, that will be assembled on the machine, will comprise of three materials. Two of the materials will be a hydrophobic nonwoven, preferably SBPP (Spundbond Polypropylene) or SMS (Spunbond-Meltblown-Spunbond). However, the hydrophobic nonwoven can also be a Through-Air Carded Web or Spunlace. Note that these two nonwoven materials may or may not be similar in basis weights. In addition, they can be different nonwoven types. For example, one may be SBPP and the other SMS. Or one may be SMS and the other Through-Air Carded Web. However, it is desired that the majority of the fibers in the nonwoven material can fuse together during ultrasonic bonding. These nonwoven materials are commercially available from Berry Global located in Charlotte, NC. The last material will be an elastic film, which is commercially available from 3M Corporation located in Minneapolis, MN.

The elastomeric material and the two substrate layers form elastomeric composites for each given section of the product chassis (i.e., the front, rear, and crotch sections). Each of the sections may be strategically constructed so as to stretch in a specific direction to help maximize the fit on the wearer from a minimum to a maximum range, thereby accommodating the varying shapes and sizes from wearer to wearer.

The front section and the rear section are both constructed so as to stretch in a machine direction (MD), while the crotch section is constructed so as to stretch in a cross direction (CD). "Cross direction (CD)", "lateral" or "transverse" and the like as used herein are used interchangeably and refer to a direction which is orthogonal to the longitudinal direction and includes directions within ±45° of the transversal direction. The product can be folded (e.g., in half) and ultrasonically bonded at the side edges to provide a disposable pull-up undergarment.

By providing different stretching characteristics for each given section, the product chassis provides a superior fitting undergarment with improved comfort, as well as reducing the risk of leakage and, in some cases, provides a much more economical disposable underwear design. For example, because the undergarment lies flat against a wearer's body in at least the three main areas (front section, rear section, and crotch section), the undergarment is about to provide a smooth contoured fit to the body, such that any gaps caused by overlapping materials are reduced or eliminated. By reducing or eliminating gaps between the garment and the body, the undergarment of the present disclosure provides improved leak resistance over prior art constructions. Additionally, the elastomeric composite provides consistent pressure and smooth contact against the leg and waist during wear when it is stretched, such that improved performance against leakage is obtained.

The smooth interface of the undergarment with the body provides improved comfort for the wearer and reduces the risk of skin irritation, particularly around the crotch and waist areas of the consumer, which are some of the most sensitive areas of the body. More specifically, the elastomeric composite design of the present disclosure eliminates the need for individual elastic strands (under high tension) and stiff materials (nonwoven & adhesive) along the leg openings, which are commonly found in current disposable underwear designs. **In** addition, the elastomeric composite design of the present disclosure allows for a wearer to participate in physical activities that may otherwise cause irritation, such as active motion of the wearer during walking or running exercises, which generally increases the rubbing action of material against the skin. The softer material and lower tension elastic provided by the undergarment of the present disclosure reduces the risk of skin irritation long the leg and crotch area of the consumer.

Furthermore, by providing different stretching characteristics for each given section, material elasticity and gasketing is increased, especially around the circumference of the leg openings, where leakage is more likely. Improvement in elasticity is due to both the type of elastomeric materials used and the way they are strategically positioned within the product. The front and back chassis of the product are designed to stretch in the machine direction (MD), which elongate in the cross-section (horizontally) along the waist, hip and thighs. The crotch section of the product is designed to stretch in the cross direction (CD), which elongate in the longitudinal direction (vertically) along the legs and crotch area. The combination of these three separate elastomeric composite sections and the manner in which they are orientated in the product, maximizes the amount of total elastic elongation. This theory has been verified in product stretch testing, as illustrated in Table 1 below:

| **Table 1 - Product Stretch Testing** | | | |
|---|---|---|---|
| **Product** | **Size** | **Maximum Leg Circumference** | **Difference** |
| Prototype 1 | L/XL | 1038 millimeters | |
| Prototype 2 | L/XL | 1092 millimeters | +54 millimeters |
| Prototype 1 | S/M | 964 millimeters | |
| Prototype 2 | S/M | 1038 millimeters | +74 millimeters |

Testing of two prototypes was performed. Prototype 1 is an elastic composite F (with MD stretch) used in the front, back and crotch sections, while Prototype 2 is an elastic composite F (with MD stretch) in the front and rear sections and elastic composite H (with CD stretch) in the crotch section, which is consistent with the disposable underwear product chassis of the present disclosure.

For testing of both prototypes, the "minimum" leg circumference was the same for similar product sizes. As shown in the table above, the elastic elongation (expansion) increased by simply replacing the elastic composite F (with MD stretch) with an elastic composite H (with CD stretch) for the crotch section. The increase was over two inches for L/XL size and nearly three inches for S/M size, using the same product dimensions.

Thus, the construction of the disposable underwear product chassis of the present disclosure allows for a decrease of the "minimum" dimension of leg circumference, while still being able to meet the original "maximum" dimension of leg circumference. By decreasing the "minimum" dimension of leg circumference, the overall elastic tension is increased and the possibility of open gaps is reduced. This improvement in "gasketing", along the leg openings, will help reduce the possibility of leakage and provide increased "sense of security" to the consumer. Consumer use testing has revealed that "tighter" leg openings in the product, with no gaps, will make most consumers feel more confident that the product will provide superior absorbency performance and will not leak.

If the elastic tension will increase along the leg openings, one may think that it will increase red marking and irritation to the skin. However, in this new product concept, that will not be the case. The elastomeric composite materials (with both MD and CD Stretch), used along the leg openings, are constructed of soft elastic film and nonwovens. The "flat" elastic film spreads out the force (elastic tension) along a larger surface area, which is much different than elastic strands, which direct the force (elastic tension) to a single point that is much smaller in surface area. This can cause red marking and irritation to the skin, if the elastic tension is too tight. If the elastic tension is too loose, the product will leak. Thus, the right balance of elastic tension and spreading out the forces along a larger surface area is required to achieve both a comfortable and anti-leak gasket at the leg area.

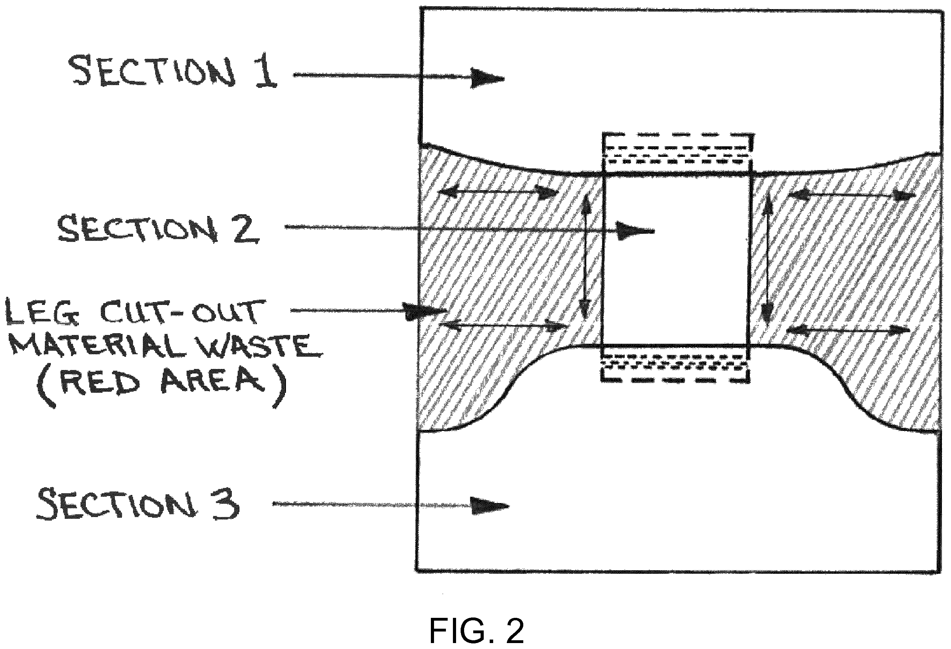

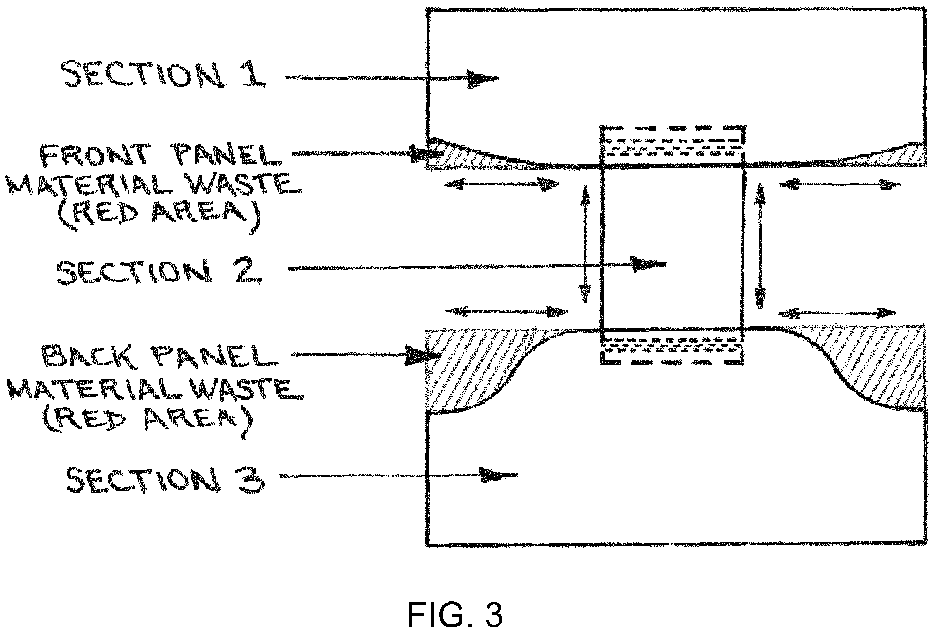

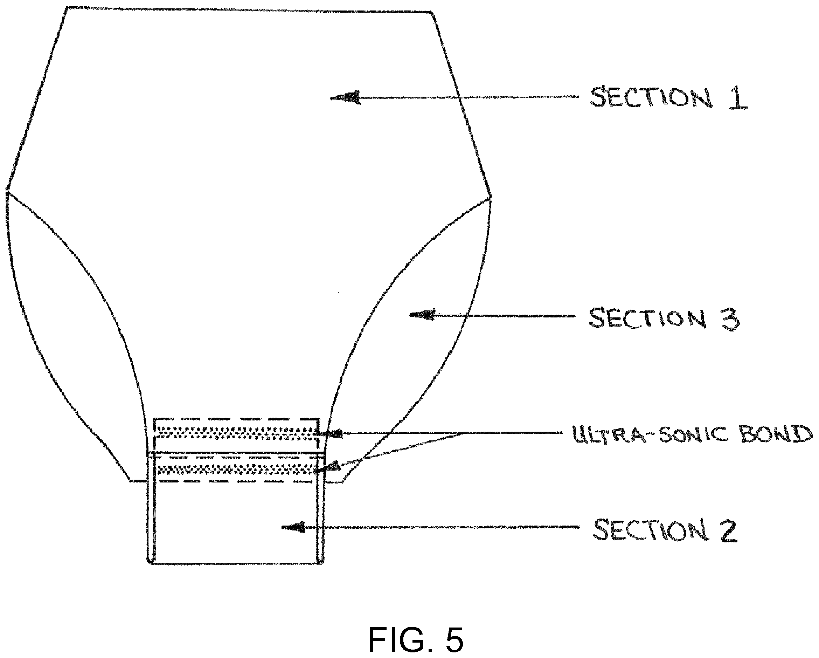

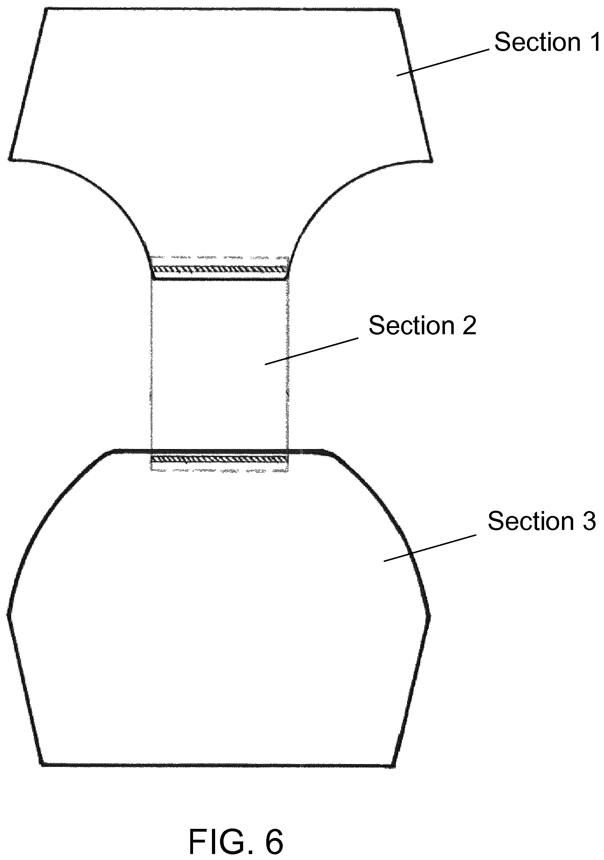

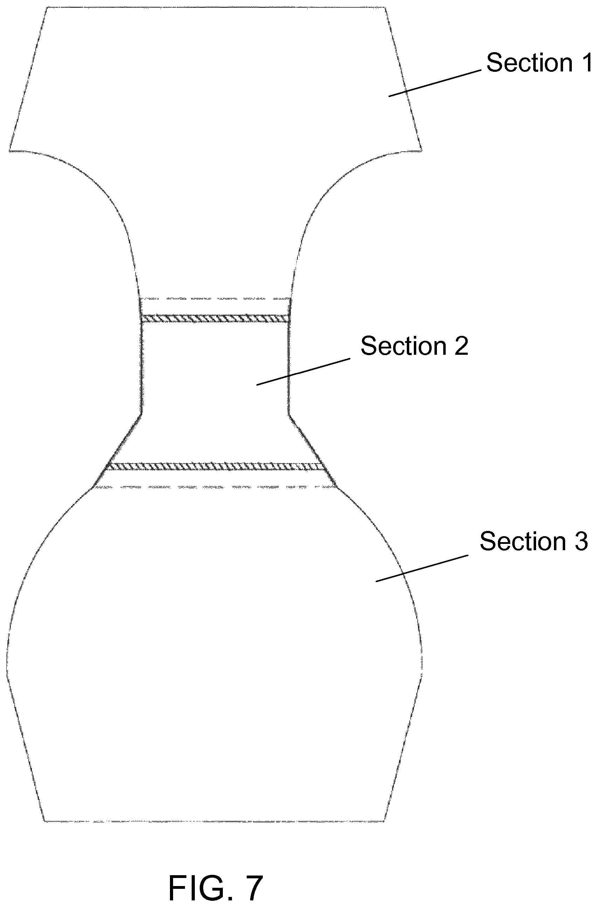

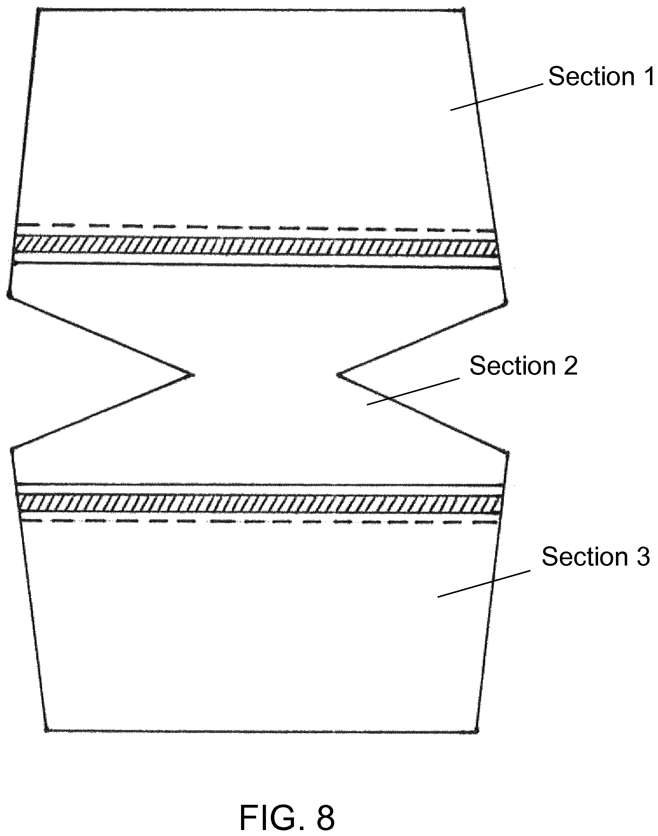

The use of at least three separately constructed sections (i.e., the front, rear, and crotch section, which are then coupled to one another to form the final undergarment product (via ultrasonic bonding or the like) minimizes the amount of waste of what could otherwise be an expensive elastomeric material. In particular, less material is necessarily required to be removed during the manufacturing process, as compared to the amount of material required to be removed if the undergarment was made entirely from single sheets of elastomeric composites. FIG. 2 is a top plan view of the disposable underwear of FIG. 1, illustrating what would constitute typical or normal material waste along leg cut-out sections (via conventional underwear design construction). FIG. 3 is a top plan view of the disposable underwear of FIG. 1, illustrating reduced material waste along leg cut-out sections. FIG. 5 is a front view of another embodiment of disposable underwear consistent with the present disclosure, illustrating the underwear in a folded and sealed state and having an anatomically-shaped design. FIG. 6 is a top plan view of the disposable underwear of FIG. 5 in an unfolded and unsealed state, illustrating reduced material waste along leg cut-out sections. FIG. 7 is a top plan view of the disposable underwear of FIG. 5 in an unfolded and unsealed state, illustrating what would constitute typical or normal material waste along leg cut-out sections (via conventional underwear design construction). FIG. 8 is a top plan view of another embodiment of disposable underwear consistent with the present disclosure, illustrating the underwear in an unfolded and unsealed state and having a boxer short shape.

As previously described, the disposable underwear consistent with the present disclosure may also contain an absorbent core for collecting bodily fluids or even excrement. For example, the disposable underwear may be used for menstrual and/or incontinence use. This would be more convenient for the consumer overall.

FIG. 4 is a top plan view of another embodiment of disposable underwear consistent with the present disclosure, illustrating the underwear including an absorbent core and insert provided within at least the crotch section, as well as a stand-up leg gather (SULP). The disposable underwear may be useful as a disposable incontinence product.

The absorbent core may generally include an absorbent material, a nonabsorbent material, and a combination thereof. For example, the absorbent core may include one or more of: "pulp only" core; "pulp & SAP" core; "pulp & SAP & tissue" core; "pulp & SAP nonwoven" core, "airlaid composite" core; "airlaid composite" core with cotton fibers; "rayon viscose" core; "rayon viscose & pulp" core; "rayon viscose & SAP" core; "rayon viscose & pulp & SAP" core; "rayon viscose & pulp & SAP & tissue" core; "tissue" core; "tissue & SAP" core; "creped tissue or paper towel" core; "creped tissue with SAP" core; "pulp & curly fiber" core; "pulp & curly fiber & SAP" core; SAP and nonwoven composite core (Pulpless); and "pulp & curly fiber & SAP & tissue" core.

The absorbent core may be comprised of multiple layers or structures. For example, as shown, the absorbent core may include at least three absorbent core structures. Optionally, more than three core structures can be used. Optionally, one or more core structures can be used, and the one or more core structures can have a variable thickness, or can have non-homogeneously-distributed constituent parts. For example, a unitary but non-homogenous core structure can have a portion that comprises fluff without SAP and another portion that comprises fluff with SAP, and optionally another portion that comprises fluff with a different proportion of SAP relative to fluff. The multicore design of the absorbent core is discussed in co-pending international application titled "Multi-Core Absorbent Article", having application no. PCT/US2016/012710, and filed January 8, 2016.

In the case of an absorbent core for menstruation, it would mainly comprise of Pulp fibers to absorb blood and other high viscosity fluids & solids. One absorbent material that would be suitable is Golden Isles fluff pulp (Grades 4865 or 4875), which have exceptional absorbency rate, anti-bacterial properties and are capable of capturing & neutralizing malodors. They are available from Georgia Pacific located in Atlanta, GA. The pulp board would be ground into fluff fibers, formed in vacuum pockets and enwrapped between a soft Top Sheet made of SBPP (Spunbond Polypropylene) Nonwoven (hydrophilic) and a soft Back Sheet made of Poly Film (fluid-impervious / hydrophobic). The Poly Film may also be microporous and breathable, if so desired. One Top Sheet Nonwoven material that would be suitable is 12 gsm SBPP from Berry Global located in Charlotte, NC. One Poly Film material that would be suitable is 0.55 mil PE/PP (Polyethylene / Polypropylene) Blend Film available from Berry Global located in Chippewa Falls, WI. Construction Adhesive will be used to hold the absorbent core system together. One Construction Adhesive that would be suitable is F-5603 olefin available from H.B. Fuller Company located in Vadnais Heights, MN.

In addition to being comprised of Pulp, the absorbent cores may also contain a small amount of SAP (Super Absorbent Polymer). This has a dual purpose. It will provide "extra" absorbent capacity and allow the product to be used as a combo Menstrual & Incontinence Pad. One SAP material that would be suitable is Favor Max 2010 available from Evonik Industries located in Greensboro, NC.

The absorbent core may also comprise of an Airlaid material, instead of Pulp fibers & SAP particles blended & formed in a vacuum pocket, as described earlier. Airlaids contain Pulp & Binder fibers, which gives it improved integrity when wet & under stress. One Airlaid material that would be suitable is Airlaid VH-145.101 (145 gsm) or VH-160.116 (160 gsm), which do not contain Superabsorbent Polymer (SAP). However, Airlaid materials may contain some SAP, in addition to Pulp & Binder fibers for dual use products (menstrual & incontinence care). Another Airlaid material that would be suitable would be VH-180.135 (180 gsm), which contains SAP. They are all available from Glatfelter Falkenhagen GmbH located in Pritzwalk, Germany.

The "shape" of the absorbent core may be symmetrical or asymmetrical, rectangular, hourglass, dog bone, etc. Usually, "hourglass shaped" cores are desired, since they are more anatomically correct. However, "wrapped" absorbent cores (Pulp/SAP) or Airlaids are usually "rectangular" in shape, to reduce material waste. Although they may be "shaped" as well for improved fit.

The absorbent core may be single layer, dual layer or even multiple layers depending on the use. Products used for overnight or require higher absorbent capacities, may use a dual core system, while products used for day or require lower absorbent capacities, may use a single core system. The single core system is usually thinner and lighter in weight.

SULG (Stand-Up Leg Gathers) or Cuffs may also be added to the product, if additional leakage performance is needed. They are mostly used in Incontinence care products, however they can be used for Feminine care products as well. The height of the SULG or Cuffs is usually higher in Incontinence care products vs. Feminine care products.

In the case of an absorbent core for incontinence, it would mainly comprise of a mixture of Pulp fibers and SAP (Super Absorbent Polymer) particles (blended together) to absorb high quantities of urine. Usually, the percentage of SAP vs. pulp is higher for incontinence care products versus menstrual care products due to the fact that SAP absorbs approximately four to five times the amount of pulp, in a gram (absorbent weight) per gram (fluid absorbed) basis.

As mentioned before, the absorbent core may be a single layer or have multiple layers depending on the absorbent capacity needed. For instance, "overnight" incontinence products may comprise of two or more layers, while "daytime" incontinence products may comprise of a single layer. If more than one absorbent layer is used, the core shape and size may be different as well. The Top Core may be mostly rectangular shaped (with round ends), while the Bottom Core may be hourglass shaped.

Also mentioned before, is the fact that the absorbent core may also comprise of an Airlaid material. In addition, the material may be a combination of Airlaid and ADL (Acquisition Distribution Layer) made of high-loft Nonwoven. One material combination that would be suitable is a Hybrid Airlaid VH-270.203 (270 gsm) available from Glatfelter Falkenhagen GmbH located in Pritzwalk, Germany. When using this material combination, it eliminates the need for an additional "cut & place" unit on the machine. Most machines already have a "cut & place" unit, along with an unwind station, to apply ADL (Acquisition Distribution Layer) on top of the absorbent core.

One "preferred embodiment" of the absorbent core would comprise of a combined Airlaid and ADL material that is "cut & placed" on top of Pulp/SAP single core. This would provide a dual absorbent core system with ADL. The combined Airlaid and ADL material would be cut shorter in length and be narrower in width vs. Pulp/SAP single core. It would also be less likely to rope, bunch & crack, thus providing improved integrity when wet and under stress.

As mentioned before, SULG or Cuffs can be added to the new product to improve leakage performance. The SULG or Cuff will comprise of hydrophobic SMS (Spunbond / Meltblown / Spunbond) Nonwoven available from Berry Global located in Charlotte, NC. The basis weight would be around 13.5 gsm.

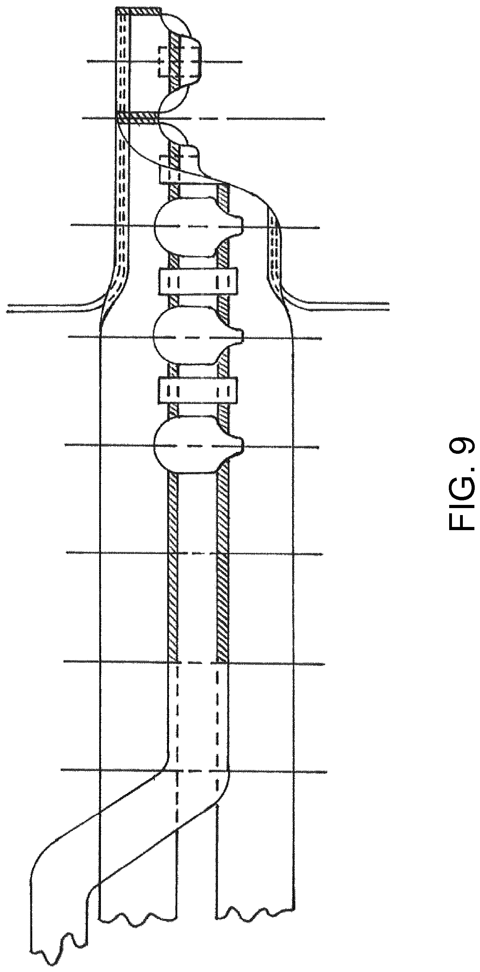

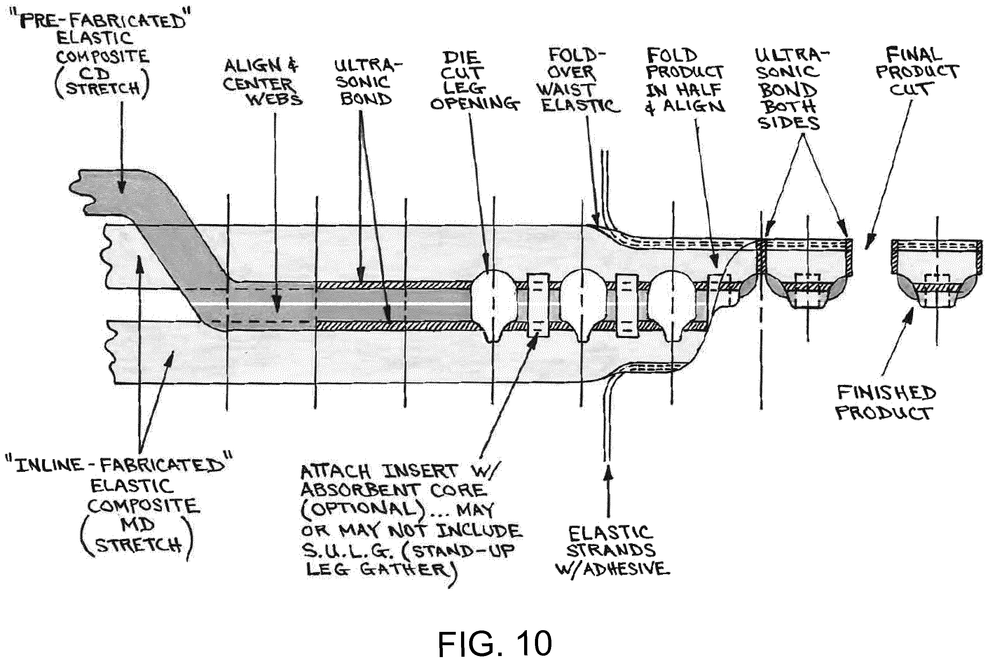

FIGS. 9 and 10 are schematic representations of a method for manufacturing a disposable underwear in accordance with the present disclosure. It should be noted that FIGS. 9 and 10 do not illustrate the manner in which the front and rear sections the Front and Rear sections of the product chassis are constructed and bonded on the machine. It is explained below, along with the rest of the manufacturing process. The steps for manufacturing a disposable underwear product chassis consistent with the present disclosure are as follows:
1. A soft nonwoven is unwound and slit in half on the machine. One nonwoven material that would be suitable is SMS (Spunbond / Meltblown / Spunbond) with Super CD Rod "embossing pattern" available from Fitesa located in Simpsonville, SC. One half of the material would be used on the "outside" of the Front Chassis and the other half on the "outside" of the Back Chassis.
2. Another nonwoven is unwound and slit in half on the machine. One nonwoven material that would be suitable is SMS (Spunbond / Meltblown / Spunbond) available from Berry Global located in Charlotte, NC. One half of the material would be used on the "inside" of the Front Chassis and the other half on the "inside" of the Back Chassis.
3. An elastic film would be unwound and slit in half on the machine. One elastic film that would be suitable is from 3M Corporation located in Minneapolis, MN. One half of the elastic material would be stretched, placed and aligned in between the nonwoven of #1 and #2 above and used in the Front Chassis. The other half of the elastic material would be stretched, placed and aligned in between the nonwoven of #1 and #2 above and used in the Back Chassis. The elongation of stretch would be around 200% - 450% in the MD (Machine Direction).
4. The three plies of material (mentioned in #1, #2 and #3 above) would be ultrasonically bonded to each other and separated at specific dimension, creating an "open gap".
5. An elastic composite would be unwound and positioned within the "open gap" mentioned in #4 above, with a slight overlap on the Front and Rear sections of the product chassis. This material is unwound in the MD (Machine Direction) on the machine and stretches in the CD (Cross Direction). One elastic composite that would be suitable would be Hexaflex 15 available from Golden Phoenix Fiberwebs, Inc. located in Taipei, Taiwan.
6. The elastic composite (mentioned in #5 above) would be ultrasonically bonded to the Front and Rear sections of the product chassis.
7. The leg openings of the product chassis would be cutout with a rotary die cutter and the scrap removed & discarded.
8. Elastic strands would be unwound, stretched and adhered to the Front and Back edges of the product chassis. Then a portion of the Front and Back edges of the product chassis would be folded over the elastic strands. The folded material can be permanently bonded to the product chassis using a heat seal. One suitable elastic strand material would be 800 Decitex from Hyosung Corporation located in Seoul, South Korea.
9. The open product would be folded in half (creating the Front and Back portions) and ultrasonically sealed at the Left & Right sides of the chassis, to create an enclosed "pull-up" product.
10. The product would be cut as the final stage to create individual "disposable underwear" pieces ready to be folded, stacked and enwrapped in a package.

By providing different stretching characteristics for each given section, the product chassis provides a superior fitting undergarment with improved comfort, as well as reducing the risk of leakage and, in some cases, provides a much more economical disposable underwear design. For example, because the undergarment lies flat against a wearer's body in at least the three main areas (front section, rear section, and crotch section), the undergarment is about to provide a smooth contoured fit to the body, such that any gaps caused by overlapping materials are reduced or eliminated. By reducing or eliminating gaps between the garment and the body, the undergarment of the present disclosure provides improved leak resistance over prior art constructions. Additionally, the elastomeric composite provides consistent pressure and smooth contact against the leg and waist during wear when it is stretched, such that improved performance against leakage is obtained.

The smooth interface of the undergarment with the body provides improved comfort for the wearer and reduces the risk of skin irritation, particularly around the crotch and waist areas of the consumer, which are some of the most sensitive areas of the body. More specifically, the elastomeric composite design of the present disclosure eliminates the need for individual elastic strands (under high tension) and stiff materials (nonwoven & adhesive) along the leg openings, which are commonly found in current disposable underwear designs. In addition, the elastomeric composite design of the present disclosure allows for a wearer to participate in physical activities that may otherwise cause irritation, such as active motion of the wearer during walking or running exercises, which generally increases the rubbing action of material against the skin. The softer material and lower tension elastic provided by the undergarment of the present disclosure reduces the risk of skin irritation long the leg and crotch area of the consumer.

The use of at least three separately constructed sections (i.e., the front, rear, and crotch section, which are then coupled to one another to form the final undergarment product (via ultrasonic bonding or the like) minimizes waste of what could otherwise be an expensive elastomeric material. In particular, less material is necessarily required to be removed during the manufacturing process, as compared to the amount of material required to be removed if the undergarment was made entirely from single sheets of elastomeric composites.

## Claims

1. A method for making a disposable absorbent product comprising the steps of:
providing a front section material in a machine direction (MD), said front section material comprising a front section elastomeric composite including an elastomeric material and configured to stretch in the machine direction upon application of a force thereto,
providing a rear section material in the machine direction (MD), said rear section material comprising a rear section elastomeric composite including an elastomeric material and configured to stretch in the machine direction upon application of a force thereto; and
providing a crotch section material in the machine direction (MD), between the front section material and the rear section material, said crotch section material comprising a crotch section elastomeric composite including an elastomeric material and configured to stretch in a cross direction (CD) substantially transverse to the machine direction upon application of a force thereto;
coupling the front and rear section materials to the crotch section material;
cutting the front and rear section materials in order to obtain a plurality of product chassis, each comprising a front section of front section material, a rear section of rear section material and a crotch section of crotch section material.

2. The method of claim 1, further comprising folding the rear section material on the front section material or folding the front section material on the rear section material, wherein the folding is performed downstream of the coupling, and upstream or downstream of the cutting, as seen in the machine direction.

3. The method of claim 1 or 2, wherein the coupling is done by ultrasonic bonding.

4. The method of any one of the previous claims, further comprising cutting leg openings extending at least in the crotch section material, and optionally extending in the front and rear section materials;
wherein preferably the cutting of leg openings is done after the step of coupling and before the step of cutting of the front and rear section materials.

5. The method of any one of the previous claims, wherein the front section and/or rear section and/or crotch section elastomeric composite comprises at least a first substrate layer and a second substrate layer, each of which comprises a nonwoven material, and a third layer comprising an elastomeric material positioned between the first and second substrate layers.

6. The method of any one of the previous claims, further comprising providing the crotch section material with an insert assembly such that it is positioned within at least a portion of the crotch section, the insert assembly comprising at least an absorbent core including a liquid-absorbing material; and/or
coupling side edges of the front section of each obtained product chassis to side edges of the rear section thereof, in order to form a garment.

7. A disposable garment comprising:
a product chassis comprising:
a front section comprising an elastomeric composite including an elastomeric material and configured to stretch in a first direction upon application of a force thereto, the front section configured to be fitted against a front, or anterior, portion of a wearer's body;
a rear section comprising an elastomeric composite including an elastomeric material and configured to stretch in the first direction upon application of a force thereto, the rear section configured to be fitted against a rear, or superior, portion of the wearer's body; and
a crotch section coupled between the front and rear sections, the crotch section comprising an elastomeric composite including an elastomeric material and configured to stretch in a second direction substantially transverse to the first direction upon application of a force thereto, the crotch section configured to be fitted against a crotch region of the wearer's body;
wherein the first direction is a machine direction and the second direction is a cross direction.

8. The disposable garment of claim 7, wherein the elastomeric composite is configured to stretch in a direction transverse to the direction in which the elastomeric material is applied to form the elastomeric composite.

9. The disposable garment of any one of the previous claims 7-8, wherein the elastomeric composite is prefabricated.

10. The disposable garment of any one of the previous claims 7-9, wherein the elastomeric composite of the front and rear sections are assembled and fabricated on a manufacturing line.

11. The disposable garment of any one of the previous claims 7-10, wherein the elastomeric composite comprises at least a first substrate layer and a second substrate layer, each of which comprises a nonwoven material;
wherein optionally the elastomeric composite comprises a third layer comprising an elastomeric film positioned between the first and second substrate layers.

12. The disposable garment of claim **11,** wherein the first and second substrate layers are coupled together by way of a seal;
wherein preferably the seal is a mechanical and heat crimp seal; and/or
wherein the first and second substrate layers are sealed to one another via an ultrasonically bonded seal to thereby form a multilayered elastomeric composite.

13. The disposable garment of any one of the claims 11-12, wherein the first substrate layer comprises a first color and the second substrate layer comprises a second color different than the first color; and/or
wherein each of the first and second substrate layers comprises more than one material;
wherein preferably at least one of the first and second substrate layers comprises a spunbond nonwoven material and the other of the first and second substrate layers comprises a through air bonded carded nonwoven material.

14. The disposable garment of any one of the previous claims 7-13, wherein the elastomeric material is a stretchable adhesive.
wherein preferably the stretch adhesive is breathable;
wherein preferably the stretch adhesive is substantially fluid impervious.

15. The disposable garment of any one of the previous claims 7-14, wherein the front and rear sections are coupled to one another by way of the crotch section;
further comprising an insert assembly positioned within at least a portion of the crotch section, the insert comprising at least an absorbent core including a liquid-absorbing material;
wherein optionally the insert assembly further comprises an acquisition layer disposed between the absorbent core and a first substrate layer;
wherein optionally the insert assembly further comprises of a polymer film barrier disposed between the absorbent core and a second substrate layer;
wherein optionally the insert assembly further comprises a top sheet applied to the acquisition layer and stand up leg gathers (SULGs) applied to the top sheet.

## Patentansprüche

1. Verfahren zum Herstellen eines Einwegabsorptionsprodukts, umfassend die Schritte:
Bereitstellen eines Frontbereichsmaterials in einer Maschinenlaufrichtung (MD), das Frontbereichsmaterial umfassend einen Frontbereichelastomerverbundstoff, der ein Elastomermaterial einschließt und konfiguriert ist, um sich in die Maschinenlaufrichtung bei Anwendung einer Kraft darauf zu dehnen, Bereitstellen eines Hinterbereichsmaterials in der Maschinenlaufrichtung (MD), das Hinterbereichsmaterial umfassend einen Hinterbereichelastomerverbundstoff, der ein Elastomermaterial einschließt und konfiguriert ist, um sich in die Maschinenlaufrichtung bei Anwendung einer Kraft darauf zu dehnen; und
Bereitstellen eines Schrittbereichsmaterials in der Maschinenlaufrichtung (MD) zwischen dem Frontbereichsmaterial und dem Hinterbereichsmaterial, das Schrittbereichsmaterial umfassend einen Schrittbereichelastomerverbundstoff, der ein Elastomermaterial einschließt und konfiguriert ist, um sich in eine Querrichtung (CD) im Wesentlichen transversal zu der Maschinenlaufrichtung bei Anwendung einer Kraft darauf zu dehnen;
Koppeln des Front- und des Hinterbereichsmaterials mit dem Schrittbereichsmaterial;
Schneiden des Front- und des Hinterbereichsmaterials, um eine Vielzahl von Produktchassis zu erhalten, jedes umfassend einen Frontbereich aus Frontbereichsmaterial, einen Hinterbereich aus Hinterbereichsmaterial und einen Schrittbereich aus Schrittbereichsmaterial.

2. Verfahren nach Anspruch 1, ferner umfassend ein Falten des Hinterbereichsmaterials auf das Frontbereichsmaterial oder das Falten des Frontbereichsmaterials auf das Hinterbereichsmaterial, wobei das Falten stromabwärts der Kopplung und stromaufwärts oder stromabwärts des Schneidens, wie in der Maschinenlaufrichtung gesehen, durchgeführt wird.

3. Verfahren nach Anspruch 1 oder 2, wobei die Kopplung durch Ultraschallbonden erfolgt.

4. Verfahren nach einem der vorhergehenden Ansprüche, ferner umfassend das Schneiden von Beinöffnungen, die sich mindestens in das Schrittbereichsmaterial erstrecken und sich optional in das Front- und das Hinterbereichsmaterial erstrecken;
wobei vorzugsweise das Schneiden von Beinöffnungen nach dem Schritt des Koppelns und vor dem Schritt des Schneidens des Front- und des Hinterbereichsmaterials erfolgt.

5. Verfahren nach einem der vorhergehenden Ansprüche, wobei der Frontbereich- und/oder der Hinterbereich- und/oder der Schrittbereichelastomerverbundstoff mindestens eine erste Substratschicht und eine zweite Substratschicht, wobei jede ein Vliesmaterial umfasst, und eine dritte Schicht, umfassend ein Elastomermaterial, das zwischen der ersten und der zweiten Substratschicht positioniert ist, umfasst.

6. Verfahren nach einem der vorhergehenden Ansprüche, ferner umfassend das Bereitstellen des Schrittbereichsmaterials mit einer Einlagenanordnung derart, dass sie innerhalb mindestens eines Abschnitts des Schrittbereichs positioniert ist, die Einlagenanordnung umfassend mindestens einen absorbierenden Kern, der ein flüssigkeitsabsorbierendes Material einschließt; und/oder
das Koppeln von Seitenrändern des Frontbereichs jedes erhaltenen Produktchassis mit Seitenrändern des Hinterbereichs davon, um ein Kleidungsstück auszubilden.

7. Einwegkleidungsstück, umfassend:
ein Produktchassis, umfassend:
einen Frontbereich, umfassend einen Elastomerverbundstoff, der ein Elastomermaterial einschließt und konfiguriert ist, um sich in eine erste Richtung bei Anwendung einer Kraft darauf zu dehnen, wobei der Frontbereich konfiguriert ist, um gegen einen Front- oder Anteriorabschnitt eines Körpers eines Trägers angepasst zu werden;
einen Hinterbereich, umfassend einen Elastomerverbundstoff, der ein Elastomermaterial einschließt und konfiguriert ist, um sich in die erste Richtung bei Anwendung einer Kraft darauf zu dehnen, wobei der Hinterbereich konfiguriert ist, um gegen einen Hinter- oder Superiorabschnitt des Körpers des Trägers angepasst zu werden; und
einen Schrittbereich, der zwischen dem Front- und dem Hinterbereich gekoppelt ist, der Schrittbereich umfassend einen Elastomerverbundstoff, der ein Elastomermaterial einschließt und konfiguriert ist, um sich in eine zweite Richtung, die im Wesentlichen transversal zu der ersten Richtung ist, bei Anwendung einer Kraft darauf zu dehnen, wobei der Schrittbereich konfiguriert ist, um gegen eine Schrittregion des Körpers des Trägers angepasst zu werden;
wobei die erste Richtung eine Maschinenlaufrichtung ist und die zweite Richtung eine Querrichtung ist.

8. Einwegkleidungsstück nach Anspruch 7, wobei der Elastomerverbundstoff konfiguriert ist, um sich in eine Richtung transversal zu der Richtung, in der das Elastomermaterial aufgebracht ist, zu dehnen, um den Elastomerverbundstoff auszubilden.

9. Einwegkleidungsstück nach einem der vorhergehenden Ansprüche 7 bis 8, wobei der Elastomerverbundstoff vorgefertigt ist.

10. Einwegkleidungsstück nach einem der vorhergehenden Ansprüche 7 bis 9, wobei der Elastomerverbundstoff des Front- und des Hinterbereichs auf einer Fertigungslinie zusammengefügt und gefertigt werden.

11. Einwegkleidungsstück nach einem der vorhergehenden Ansprüche 7 bis 10, wobei der Elastomerverbundstoff mindestens eine erste Substratschicht und eine zweite Substratschicht umfasst, die jede ein Vliesmaterial umfasst;
wobei optional der Elastomerverbundstoff eine dritte Schicht umfasst, umfassend eine Elastomerfolie, die zwischen der ersten und der zweiten Substratschicht positioniert ist.

12. Einwegkleidungsstück nach Anspruch 11, wobei die erste und die zweite Substratschicht mittels einer Versiegelung miteinander gekoppelt sind;
wobei vorzugsweise die Versiegelung eine mechanische und eine Wärme-Crimpversiegelung ist; und/oder
wobei die erste und die zweite Substratschicht über eine ultraschallgebondete Versiegelung miteinander versiegelt sind, um dadurch einen mehrschichtigen Elastomerverbundstoff auszubilden.

13. Einwegkleidungsstück nach einem der Ansprüche 11 bis 12, wobei die erste Substratschicht eine erste Farbe umfasst und die zweite Substratschicht eine zweite Farbe, die sich von der ersten Farbe unterscheidet, umfasst; und/oder
wobei jede der ersten und der zweiten Substratschicht mehr als ein Material umfasst;
wobei vorzugsweise mindestens eine der ersten und der zweiten Substratschicht ein Spinnvliesmaterial umfasst und die andere der ersten und der zweiten Substratschicht ein durchluftgebondetes kardiertes Vliesmaterial umfasst.

14. Einwegkleidungsstück nach einem der vorhergehenden Ansprüche 7 bis 13, wobei das Elastomermaterial ein dehnbarer Klebstoff ist,
wobei vorzugsweise der dehnbare Klebstoff atmungsaktiv ist;
wobei vorzugsweise der dehnbare Klebstoff im Wesentlichen fluidundurchlässig ist.

15. Einwegkleidungsstück nach einem der vorhergehenden Ansprüche 7 bis 14, wobei der Front- und der Hinterbereich über den Schrittbereich miteinander gekoppelt sind;
ferner umfassend eine Einlagenanordnung, die innerhalb mindestens eines Abschnitts des Schrittbereichs positioniert ist, die Einlagenanordnung umfassend mindestens einen absorbierenden Kern, der ein flüssigkeitsabsorbierendes Material einschließt;
wobei optional die Einlagenanordnung ferner eine Akquisitionsschicht, die zwischen dem absorbierenden Kern und einer ersten Substratschicht arrangiert ist, umfasst;
wobei optional die Einlagenanordnung ferner eine Polymerfolienbarriere, die zwischen dem absorbierenden Kern und einer zweiten Substratschicht arrangiert ist, umfasst;
wobei optional die Einlagenanordnung ferner eine obere Lage, die auf die Aufnahmeschicht aufgebracht ist, und Stand-Up-Leg-Gathers (SULGs), die auf die obere Lage aufgebracht sind, umfasst.

## Revendications

1. Procédé pour la fabrication d'un produit absorbant jetable, comprenant les étapes consistant à :
fournir un matériau de section avant dans un sens machine (MD), ledit matériau de section avant comprenant un composite élastomère de section avant comportant un matériau élastomère et conçu pour s'étirer dans le sens machine lors de l'application d'une force à celui-ci, fournir un matériau de section arrière dans le sens machine (MD), ledit matériau de section arrière comprenant un composite élastomère de section arrière comportant un matériau élastomère et conçu pour s'étirer dans le sens machine lors de l'application d'une force à celui-ci ; et
fournir un matériau de section d'entrejambe dans le sens machine (MD), entre le matériau de section avant et le matériau de section arrière, ledit matériau de section d'entrejambe comprenant un composite élastomère de section d'entrejambe comportant un matériau élastomère et conçu pour s'étirer dans un sens transversal (CD) sensiblement transversal au sens machine lors de l'application d'une force à celui-ci ;
accoupler des matériaux de sections avant et arrière au matériau de section d'entrejambe ;
découper les matériaux de sections avant et arrière afin d'obtenir une pluralité de châssis de produit, chacun comprenant une section avant de matériau de section avant, une section arrière de matériau de section arrière et une section d'entrejambe de matériau de section d'entrejambe.

2. Procédé selon la revendication 1, comprenant en outre le pliage du matériau de section arrière sur le matériau de section avant ou le pliage du matériau de section avant sur le matériau de section arrière, dans lequel le pliage est effectué en aval de l'accouplement, et en amont ou en aval de la découpe, vu dans le sens machine.

3. Procédé selon la revendication 1 ou 2, dans lequel l'accouplement est réalisé par liage par ultrasons.

4. Procédé selon l'une quelconque des revendications précédentes, comprenant en outre la découpe d'ouvertures de jambes s'étendant au moins dans le matériau de section d'entrejambe, et s'étendant éventuellement dans les matériaux de sections avant et arrière ;
dans lequel, de préférence, la découpe des ouvertures de jambes est réalisée après l'étape d'accouplement et avant l'étape de découpe des matériaux de sections avant et arrière.

5. Procédé selon l'une quelconque des revendications précédentes, dans lequel le composite élastomère de section avant et/ou de section arrière et/ou de section d'entrejambe comprend au moins une première couche de substrat et une seconde couche de substrat, chacune comprenant une matériau non tissé, et une troisième couche comprenant un matériau élastomère positionné entre les première et seconde couches de substrat.

6. Procédé selon l'une quelconque des revendications précédentes, comprenant en outre la fourniture du matériau de section d'entrejambe avec un ensemble insert de telle sorte qu'il est positionné à l'intérieur d'au moins une partie de la section d'entrejambe, l'ensemble insert comprenant au moins une âme absorbante comportant un matériau absorbant les liquides ; et/ou
l'accouplement de bords latéraux de la section avant de chaque châssis de produit obtenu à des bords latéraux de la section arrière de celui-ci, afin de former un vêtement.

7. Vêtement jetable comprenant :
un châssis de produit comprenant :
une section avant comprenant un composite élastomère comportant un matériau élastomère et conçue pour s'étirer dans un premier sens lors de l'application d'une force à celle-ci, la section avant étant conçue pour être ajustée contre une partie avant, ou antérieure, du corps d'un porteur ;
une section arrière comprenant un composite élastomère comportant un matériau élastomère et conçue pour s'étirer dans le premier sens lors de l'application d'une force à celle-ci, la section arrière étant conçue pour être ajustée contre une partie arrière, ou supérieure, du corps du porteur ; et
une section d'entrejambe accouplée entre les sections avant et arrière, la section d'entrejambe comprenant un composite élastomère comportant un matériau élastomère et étant conçue pour s'étirer dans un second sens sensiblement transversal au premier sens lors de l'application d'une force à celle-ci, la section d'entrejambe étant conçue pour être ajustée contre une région d'entrejambe du corps du porteur ;
dans lequel le premier sens est un sens machine et le second sens est un sens transversal.

8. Vêtement jetable selon la revendication 7, dans lequel le composite élastomère est conçu pour s'étirer dans un sens transversal au sens dans lequel le matériau élastomère est appliqué pour former le composite élastomère.

9. Vêtement jetable selon l'une quelconque des revendications précédentes 7 à 8, dans lequel le composite élastomère est préfabriqué.

10. Vêtement jetable selon l'une quelconque des revendications précédentes 7 à 9, dans lequel le composite élastomère des sections avant et arrière est assemblé et fabriqué sur une chaîne de fabrication.

11. Vêtement jetable selon l'une quelconque des revendications précédentes 7 à 10, dans lequel le composite élastomère comprend au moins une première couche de substrat et une seconde couche de substrat, chacune comprenant un matériau non tissé ;
dans lequel le composite élastomère comprend éventuellement une troisième couche comprenant un film élastomère positionné entre les première et seconde couches de substrat.

12. Vêtement jetable selon la revendication 11, dans lequel les première et seconde couches de substrat sont accouplées ensemble au moyen d'un scellement ;
dans lequel, de préférence, le scellement est un scellement mécanique et à sertissage thermique ; et/ou
dans lequel les première et seconde couches de substrat sont scellées l'une à l'autre par l'intermédiaire d'un scellement lié par ultrasons pour former ainsi un composite élastomère multicouche.

13. Vêtement jetable selon l'une quelconque des revendications 11 à 12, dans lequel la première couche de substrat comprend une première couleur et la seconde couche de substrat comprend une seconde couleur différente de la première couleur ; et/ou
dans lequel chacune parmi les première et seconde couches de substrat comprend plus d'un matériau ;
dans lequel, de préférence, au moins l'une parmi les première et seconde couches de substrat comprend un matériau non tissé filé-lié et l'autre parmi les première et seconde couches de substrat comprend un matériau non tissé cardé lié par air traversant.

14. Vêtement jetable selon l'une quelconque des revendications précédentes 7 à 13, dans lequel le matériau élastomère est un adhésif extensible.
dans lequel, de préférence, l'adhésif extensible est respirant ;
dans lequel, de préférence, l'adhésif extensible est sensiblement imperméable aux fluides.

15. Vêtement jetable selon l'une quelconque des revendications précédentes 7 à 14, dans lequel les sections avant et arrière sont accouplées l'une à l'autre par le biais de la section d'entrejambe ;
comprenant en outre un ensemble insert positionné à l'intérieur d'au moins une partie de la section d'entrejambe, l'insert comprenant au moins une âme absorbante comportant un matériau absorbant les liquides ;
dans lequel, éventuellement, l'ensemble insert comprend en outre une couche d'acquisition disposée entre l'âme absorbante et une première couche de substrat ;
dans lequel, éventuellement, l'ensemble insert comprend en outre une barrière de film polymère disposée entre l'âme absorbante et une seconde couche de substrat ;
dans lequel, éventuellement, l'ensemble insert comprend en outre une feuille supérieure appliquée sur la couche d'acquisition et des fronces de jambe dressées (SULG) appliquées sur la feuille supérieure.
